# EUROPEAN PATENT SPECIFICATION

(11) **EP 2 831 287 B1**
(45) Date of publication and mention of the grant of the patent: **08.03.2017**
(21) Application number: 13723197.3
(22) Date of filing: 26.03.2013
(51) Int. Cl.: C12R 1/01, A61K 35/745

(54) **COMPOSITION BASED ON STRAINS OF BIFIDOBACTERIUM LONGUM BACTERIA CAPABLE OF HELPING TO PROLONG LIFE**
ZUSAMMENSETZUNG AUF BASIS VON BIFIDOBACTERIUM-LONGUM-BAKTERIENSTÄMMEN ZUR LEBENSVERLÄNGERUNG
COMPOSITION CONTENANT DES SOUCHES DE BACTÉRIES BIFIDOBACTERIUM LONGUM CAPABLE D'AIDER À PROLONGER LA VIE

(30) Priority: 26.03.2012 IT MI20120471
(43) Date of publication of application: 04.02.2015
(73) Proprietor: Mogna, Giovanni, 28100 Novara (NO) (IT)
(72) Inventor: MOGNA, Giovanni, 28100 Novara (IT); DRAGO, Lorenzo, 20900 Monza (IT)
(74) Representative: Hoffmann Eitle
(86) International application number: PCT/IB2013/000518
(87) International publication number: WO 2013/144701

(56) References cited:
- EP-A1- 2 233 011
- IE-A1- 990 033
- US-B1- 6 306 638
- MOGNA LUCA ET AL: "Selenium and zinc internalized by Lactobacillus buchneri Lb26 (DSM 16341) and Bifidobacterium lactis Bb1 (DSM 17850): improved bioavailability using a new biological approach", JOURNAL OF CLINICAL GASTROENTEROLOGY UNITED STATES, USA, vol. 46 Suppl, 1 October 2012 (2012-10-01) , pages S41-S45, XP009164978, ISSN: 1539-2031
- DRAGO LORENZO ET AL: "Cultivable and pyrosequenced fecal microflora in centenarians and young subjects", JOURNAL OF CLINICAL GASTROENTEROLOGY UNITED STATES, USA, vol. 46 Suppl, 1 October 2012 (2012-10-01) , pages S81-S84, XP009164979, ISSN: 1539-2031
- TIMMERMAN H M ET AL: "Monostrain, multistrain and multispecies probiotics-A comparison of functionality and efficacy", INTERNATIONAL JOURNAL OF FOOD MICROBIOLOGY, ELSEVIER BV, NL, vol. 96, no. 3, 15 November 2004 (2004-11-15), pages 219-233, XP004580747, ISSN: 0168-1605, DOI: 10.1016/J.IJFOODMICRO.2004.05.012
- WARNAKULASURIYA MARY ANN DIPIKA BIN FERNANDO ET AL: "The effect of rice fibre fractions on the growth of co-cultures of probiotics", JOURNAL OF FOOD SCIENCE AND TECHNOLOGY, vol. 48, no. 1, 8 November 2010 (2010-11-08), pages 14-25, XP055065918, ISSN: 0022-1155, DOI: 10.1007/s13197-010-0147-5
- CAZZOLA M., TOMPKINS T., MATERA M.: "Immunomodulatory impact of a synbioticin TH1 and TH2 models of infection", THERAPEUTIC ADVANCES IN RESPIRATORY DISEASE, vol. 0, no. 0, 2010, pages 1-13, XP002698490, DOI: 10.1177/1753465810379009

## Description

The present invention relates to a composition based on bacteria belonging to the species *Bifidobacterium longum* capable of helping to prolong life and promote good kidney and/or intestinal function, as well as assuring the body's well-being.

It is well known that intestinal microflora (microbiota) plays an essential role in the body's well-being and in human health. It is likewise known that intestinal microflora is characterized by the presence of a delicate balance between a complex population of pathogenic bacterial species and an equally complex population of bacteria that perform activity that is beneficial and essential for the body. To date, many efforts have been made to get to know and understand the complex microbiota system in order to determine which populations of pathogens colonize the gastrointestinal tract and which bacterial species are beneficial for the body. Above all, many efforts have been made to understand and determine what factors contribute to modifying the complex balance within the population of pathogenic species, within the population of bacteria that bring benefits to the body and between these two populations. Therefore, there remains a need to be able to identify and select, within the population of bacterial species that are beneficial for the body, the prevalent bacterial species which are present in the complex microbiota system; once isolated, it is likewise necessary to be able to determine their effects on the body in order to be able to prepare specific pharmaceutical compositions or supplement products or medical devices or food compositions.

The Applicant conducted an experimental phase in which it identified, selected, isolated and characterized some bacterial strains which on being tested revealed excellent and unexpected health-promoting properties in terms of helping to prolong life and promoting good intestinal function as well as assuring the body's well-being and a state of health that is prolonged over time.

The present invention relates to a bacterial strain belonging to the species *Bifidobacterium longum,* as claimed in the appended claim.

The present invention relates to a process for preparing cultures of bacteria belonging to the species *Bifidobacterium longum,* wherein said process comprises a step in which at least two bacterial strains, belonging to the species *Bifidobacterium longum -* selected from among DLBL 07, DLBL 08 and DLBL 10, as described below - are made to grow together synergistically in the same culture substrate, as claimed in the appended claim.

The present invention further describes a pharmaceutical composition or a medical device or a supplement product or a food composition or a cosmetic composition comprising at least two bacterial strains belonging to the same species *Bifidobacterium longum,* obtained via a process of bacterial co-culture, as claimed in the appended claim.

Further preferred embodiments of the present invention will be set forth and illustrated in the detailed description that follows, without intending to limit in any way the scope of the present invention.

The Applicant engaged in intense research activity, during which it identified, selected, isolated and characterized the following bacterial strains, which form the subject matter described in the present invention, as claimed in the claim 1.

The following bacterial strains were deposited by the company Probiotical SpA, Via Mattei, 3 -28100 Novara (NO) Italy with the *DSMZ-Deutsche Sammlung von Mikro-organismen und Zellkulturen* GmbH, Germany on 16.02.2012, in accordance with the Budapest Treaty:
1) *Bifidobacterium longum* DLBL 07 = DSM 25669
*2) Bifidobacterium longum* DLBL 08 = DSM 25670
3) *Bifidobacterium longum* DLBL 09 = DSM 25671
*4) Bifidobacterium longum* DLBL 10 = DSM 25672
*5) Bifidobacterium longum* DLBL 11 = DSM 25673
*6) Bifidobacterium longum* DLBL 12 = DSM 25674
*7) Bifidobacterium longum* DLBL 13 = DSM 25675
8) *Bifidobacterium longum* DLBL 14 = DSM 25676
9) *Bifidobacterium longum* DLBL 15 = DSM 25677
10) *Bifidobacterium longum* DLBL 16 = DSM 25678
11) *Bifidobacterium longum* DLBL 17 = DSM 25679

The following bacterial strain was deposited by the company Probiotical SpA, Via Mattei, 3 -28100 Novara (NO) Italy with the *DSMZ-Deutsche Sammlung von Mikro-organismen und Zellkulturen* GmbH, Germany on 24.02.2012, in accordance with the Budapest Treaty:
*12) Bifidobacterium longum* DLBL 18 = DSM 25708

The following bacterial strains were deposited by the company Probiotical SpA, Via Mattei, 3 -28100 Novara (NO) Italy with the *DSMZ-Deutsche Sammlung von Mikro-organismen und Zellkulturen* GmbH, Germany on 01.03.2012, in accordance with the Budapest Treaty:
13) *Bifidobacterium longum* DLBL 19 = DSM 25717
14) *Bifidobacterium longum* DLBL 20 = DSM 25718

The above-mentioned bacterial strains are validly employed to prepare a pharmaceutical composition or a medical device or a supplement product or a food composition or a cosmetic composition (hereinafter briefly "the compositions ") as disclosed hereunder. All of these compositions comprise or, alternatively, consist of a bacterial mixture which comprises or, alternatively, consists of at least two bacterial strains belonging to the species *Bifidobacterium longum.* Said mixture which comprises or, alternatively, consists of these bacterial strains is prepared with a process of synergistic "co-culture" of individual bacterial strains belonging to the same species, *B. longum.* Said strains are selected from the group comprising or, alternatively, consisting of the strains indicated as (1), (2) and (4), as described below.

The Applicant found it useful to carry out fermentation by means of a process of synergistic "co-culture" of individual bacterial strains belonging to the same species, *B. longum,* using a number of strains equal to or greater than 2. The co-culture is carried out using techniques and equipment known to the person skilled in the art, who is capable of selecting the culture media, the sources of nitrogen and carbon, as well as the operating conditions for cell fermentation and multiplication. The number of strains used in the co-culture process is comprised from 2 to 14 strains; preferably, said strains are selected from among those indicated above as (1) to (14). The number of the strains used in the co-culture process ranges from 2 to 14 (for example, 2 strains or 3 strains or 4 strains or 5 strains or 6 strains or 7 strains or 8 strains or 9 strains or 10 strains or 11 strains or 12 strains or 13 strains or 14 strains). The strains used in a process of synergistic "co-culture" of bacteria of the same species, *B*. *longum,* are selected from among those belonging to the species *B*. *longum.* Preferably, said strains are those indicated above as (1) to (14). It was observed that the individual bacterial strains belonging to the same species *B. longum* advantageously synergize with one another during the bacterial "co-culture". Practically speaking, fermentation carried out via "co-culture" of bacteria of the same species, *B*. *longum* (i.e. carried out using more than 2 strains, for example among those indicated as (1) to (14)), produces a total number of cells that is greater than the total number of bacterial cells obtained by fermentation using a single strain.

Moreover, during the bacterial co-culture, metabolites are produced which characterize the culture substrate from both a qualitative and quantitative viewpoint, as well as from the viewpoint of the intrinsic properties of the culture substrate itself. The process of synergistic "co-culture" of bacteria of the same species, *B. longum,* in reality produces enhancing effects both during production (fermentation) and during use in humans (in the intestine in particular). The enhancing effects refer not only to the quantity and biological activity of the bacterial cells obtained, but also to the quantity and quality of the bacterial metabolites.

Therefore, all the compositions of the present invention are prepared using the bacterial strains produced via fermentation carried out with a process of "co-culture" of bacteria of the same species, *B. longum*; preferably, said strains are those indicated as (1) to (14). For this reason, the compositions distinguish themselves advantageously from those present in the prior art.

All of the bacterial strains prepared via co-culture reveal excellent and unexpected health-promoting properties in terms of helping to prolong life and promote good kidney and/or intestinal function, as well as assuring the body's well-being and a state of health that is prolonged over time.

Advantageously, the bacterial strains of the present invention are capable of producing cytokines. The analysis of the individual cytokines secreted demonstrated the ability of the bacterial strains to induce an increase both in the pro-inflammatory cytokine IFN-gamma and the anti-inflammatory/regulating cytokine IL-10. Moreover, the Th1/Th2 ratio is greater than 1. The bacterial mixture present in the composition is characterized by a number of bacterial strains which is capable of modulating the Th1/Th2 ratio in an optimal manner because each of said strains has a tendency of its own to induce an increase in the pro-inflammatory cytokine IFN-gamma or the anti-inflammatory/regulating cytokine IL-10.

In one embodiment of the present invention, the process of preparing a bacterial mixture comprises co-culturing at least two bacterial strains selected from the group consisting of:
1) *Bifidobacterium longum* DLBL 07 = DSM 25669
2) *Bifidobacterium longum* DLBL 08 = DSM 25670
*4) Bifidobacterium longum* DLBL 10 = DSM 25672

In one embodiment of the present invention the process of preparing a bacterial mixture comprises co-culturing the following five bacterial strains (5-strain mixture):
- *Bifidobacterium longum* DLBL 07 = DSM 25669
- *Bifidobacterium longum* DLBL 08 = DSM 25670
- *Bifidobacterium longum* DLBL 09 = DSM 25671
- *Bifidobacterium longum* DLBL 10 = DSM 25672
- *Bifidobacterium longum* DLBL 11 = DSM 25673.

In one embodiment of the present invention the process of preparing a bacterial mixture comprises co-culturing the following four bacterial strains (4-strain mixture):
- *Bifidobacterium longum* DLBL 07 = DSM 25669
- *Bifidobacterium longum* DLBL 08 = DSM 25670
- *Bifidobacterium longum* DLBL 09 = DSM 25671
- *Bifidobacterium longum* DLBL 11 = DSM 25673.

In one embodiment of the present invention the process of preparing a bacterial mixture comprises co-culturing the following three bacterial strains (3-strain mixture (a)):
- *Bifidobacterium longum* DLBL 07 = DSM 25669
- *Bifidobacterium longum* DLBL 08 = DSM 25670
- *Bifidobacterium longum* DLBL 11 = DSM 25673.

The present invention also describes the process of preparing a bacterial mixture comprising co-culturing the following three bacterial strains (3-strain mixture (b)):
- *Bifidobacterium longum* DLBL 09 = DSM 25671
- *Bifidobacterium longum* DLBL 10 = DSM 25672
- *Bifidobacterium longum* DLBL 11 = DSM 25673.

The above-mentioned mixtures (mixtures of 5 strains, 4 strains and 3 strains (a) and (b)) have a concentration (bacterial count) comprised from 1x10⁸ to 1x10¹¹ CFU/g of mixture, preferably comprised from 1x10⁹ to 1x10¹⁰ CFU/g of mixture. In the 5-strain mixture, each strain is present in a ratio by weight of 1:1:1:1:1. Each individual strain has a concentration (bacterial count) comprised from 1x10⁸ to 1x10¹¹ CFU/g of bacteria, preferably comprised from 1x10⁹ to 1x10¹⁰ CFU/g of bacteria. In the 4-strain mixture, each strain is present in a ratio by weight of 1:1:1:1. Each individual strain has a concentration (bacterial count) comprised from 1x10⁸ to 1x10¹¹ CFU/g of bacteria, preferably comprised from 1x10⁹ to 1x10¹⁰ CFU/g of bacteria. In the 3-strain mixtures (a) and (b), each strain is present in a ratio by weight of 1:1:1.

Each individual strain has a concentration (bacterial count) comprised from 1x10⁸ to 1x10¹¹ CFU/g of bacteria, preferably comprised from 1x10⁹ to 1x10¹⁰ CFU/g of bacteria.

In the context of the present invention, the bacterial strains belonging to the species *Bifidobacterium longum* can be present in the above-mentioned mixtures and thus in said compositions in the form of live cells and/or dead cells and/or as a metabolite and/or as a cellular derivative and/or cellular or enzymatic component thereof.

The compositions can be administered to all categories of people without any limitation in order to help to prolong the life of an individual; to slow down and/or combat and/or reduce biological aging processes, for example physical and/or skin aging; to reduce aging processes that lead to loss of memory or visual memory and/or ability to concentrate; to inhibit the production of bacteroides by means of a non-specific mechanism of inhibition (production of metabolites) and/or a specific one (production of bacteriocins); to stimulate the production of butyric *Clostridia* capable of producing butyrates able to inhibit phenomena leading to the onset of colitis, ulcerative colitis, IBD (Inflammatory Bowel Disease) and Crohn's disease; to inhibit and/or reduce the production of enterobacteria belonging to the *Enterobacteriaceae* family, in particular to reduce the number of enterobacteria normally present in a microbiota; to modify the balance present in intestinal microflora in order to make the species *Bifidobacterium longum* prevail; to positively influence antioxidant activity and immunomodulating activity with the production of cytokines; to reduce the content of toxic metabolites produced by or derived from protein degradation, thereby helping or improving kidney function.

The present invention describes a pharmaceutical or cosmetic composition or a medical device or a supplement product or a food composition comprising a mixture of strains bacteria comprising at least two bacterial strains indicated as (1) to (14) obtained with the co-culture process. Said composition has valid application for use in a treatment to slow down and/or combat and/or reduce biological processes of physical and skin aging; or for use in a treatment to slow down and/or combat and/or reduce biological aging processes which lead to loss of memory and/or ability to concentrate. Said mixture of bacterial strains can be selected from among the 5-strain mixture, 4-strain mixture or 3-strain mixture (a) or (b) having the characteristics set forth above.

The present invention describes a pharmaceutical or cosmetic composition or a medical device or a supplement product or a food composition comprising a mixture of bacterial strains comprising at least two bacterial strains indicated as (1) to (14) obtained with the co-culture process. Said composition has valid application for use in a treatment to inhibit the production of bacteroides by means of a non-specific mechanism of inhibition which involves the production of metabolites by said bacteria and/or by means of a specific mechanism which involves the production of bacteriocins by said bacteria; or for use in a treatment to stimulate the production of butyric *Clostridia* capable of producing butyrates to combat the onset of colitis, ulcerative colitis, an inflammation of the intestine or of the gastrointestinal tract or Crohn's disease. Said mixture of bacterial strains can be selected from among the 5-strain mixture, the 4-strain mixture or the 3-strain mixture (a) or (b) having the characteristics described above.

The present invention describes a pharmaceutical or cosmetic composition or a medical device or a supplement product or a food composition comprising a mixture of bacterial strains comprising at least two bacterial strains indicated as (1) to (14) obtained with the co-culture process. Said composition has valid application for use in a treatment to reduce the content of toxic metabolites produced or derived from protein degradation in order to improve kidney function. Said mixture of bacterial strains can be selected from among the 5-strain mixture, the 4-strain mixture or the 3-strain mixture (a) or (b) having the characteristics described above.

The present invention describes a pharmaceutical or cosmetic composition or a medical device or a supplement product or a food composition comprising a mixture of bacterial strains comprising at least two bacterial strains indicated as (1) to (14) obtained with the co-culture process. Said composition has valid application for use in an antioxidant or immunomodulating treatment with the production of cytokines. Said mixture of bacterial strains can be selected from among the 5-strain mixture, the 4-strain mixture or the 3-strain mixture (a) or (b) having the characteristics described above.

Advantageously, all the compositions have valid application in maintaining a good and efficient kidney function and/or in the treatment of kidney function in subjects who suffer from kidney diseases and/or disorders. The disorders connected to an alteration in kidney function or kidney failure (often present in the elderly) result in an increase in toxic metabolites due to protein degradation. These disorders are mainly linked to cardiopathies and encephalopathies.

The bacterial strains belonging to the species *Bifidobacterium longum,* disclosed in the present invention, can help contribute to prolonging the life of a human being since said strains can intervene in a significant manner thanks to their proteasome (UPS= ubiquitin-proteasome-system). The action of the proteasome makes it possible to combat the biological phenomena which lead to aging, thus preserving the physical and/or mental state of a human being.

Advantageously, all the compositions comprise or, alternatively, consist of a variable number of bacterial strains, belonging to the species *B*. *longum,* comprised from 2 to 14 (for example, 2 strains or 3 strains or 4 strains or 5 strains or 6 strains or 7 strains or 8 strains or 9 strains or 10 strains or 11 strains or 12 strains or 13 strains or 14 strains) which are produced via a process of "co-culture" of individual bacterial strains belonging to the same species *B*. *longum*; preferably, said strains are selected from among those indicated above as (1) to (14).

Advantageously, the compositions, besides comprising a mixture of bacterial strains selected from among the 5-strain mixture or the 4-strain mixture or the 3-strain mixture (a) or (b), can comprise N-acetylcysteine (NAC) as such or a substance based on N-acetylcysteine (NAC) or a derivative thereof in association with at least two bacterial strains produced via a process of "co-culture" of individual bacterial strains belonging to the same species *B*. *longum*; preferably, said strains are selected from among those indicated above as (1) to (14).

In this case, there is a further improvement in kidney function due also to the decrease in toxic metabolites resulting from altered protein degradation and produced by decarboxylation of amino acids (biogenic amines produced by *E.coli*). Therefore, in the present invention it is envisaged to use N-acetylcysteine, both in free form and in microencapsulated gastroprotected form (from 10 to 1000 mg/die), which has a mechanical barrier effect in hindering the ability of *E.coli* to adhere to the intestinal wall. Furthermore, N-acetylcysteine stimulates the production of glutathione and thus has an antioxidant ability. The compositions are capable of preserving proteasome activity. For this reason they can be validly administered to people to help prolong their life.

The mixture of bacterial strains selected from among the 5-strain mixture or the 4-strain mixture or the 3-strain mixture (a) or (b) is present in the composition in an amount comprised from 0.1 to 50% by weight, preferably from 0.5 to 25% by weight; even more preferably from 1 to 15% by weight, relative to the total weight of the composition. However, said percentage relative to the total weight of the composition depends on the product category of the composition it is intended to prepare. For example, in a capsule the amount of said bacteria is preferably greater than 40%.

The compositions contain bacteria having a concentration comprised from 1x10⁶ to 1x10¹¹ CFU /g, preferably from 1x10⁸ to 1x10¹⁰CFU/g.

The compositions can contain bacteria in a concentration comprised from 1x10⁶ to 1x10¹¹ CFU/dose, preferably from 1x10⁸ to 1x10¹⁰ CFU/dose. The dose can be comprised from 0.2 to 10 g; for example, it is 0.25 g, 1 g, 3 g, 5 g or 7 g.

The bacteria used in the present invention can be in solid form, in particular in powder, dehydrated powder, spray or lyophilized form.

The food composition or supplement product or medical device or pharmaceutical composition can further comprise some prebiotic fibres and carbohydrates having a bifidogenic action, such as, for example, inulin, fructo-oligosacharides (FOS), galacto- and trans-galactooligosaccharides (GOS and TOS), gluco-oligosaccharides (GOSa), xylo-oligosaccharides (XOS), chitosan oligosaccharides (COS), soy oligosaccharides (SOS), isomalto-oligosaccharides (IMOS), resistant starch, pectin, psyllium, arabinogalactan, glucomannan, galactomannan, xylan, lactosucrose, lactulose, lactitol and various other types of gums, preferably tara gum, acacia, carob, oat or bamboo, citrus fibres and, in general, fibres containing a soluble and an insoluble portion, in a variable ratio to each other.

Advantageously, said fibre is selected from the group comprising FOS, inulin and citrus fibres, preferably in a ratio by weight of from 1:3 to 3:1.

The amount of the prebiotic fibres and/or carbohydrates having a bifidogenic action, if present, is comprised from 0.5 to 75% by weight, preferably from 1% to 40% and even more preferably from 2 to 20% relative to the total weight of the composition. In this case one has a composition or supplement with symbiotic activity.

The compositions can further comprise one or more physiologically acceptable additives or excipients as well as comprising other active ingredients and/or components, such as vitamins, minerals, bioactive peptides, substances having antioxidant, hypocholesterolemizing, hypoglycemizing, anti-inflammatory or sweetening activity in an amount by weight generally comprised from 0.001% to 10% by weight, preferably from 0.5% to 5%, depending in any case on the type of active component and the recommended daily allowance thereof, if defined, relative to the total weight of the composition.

The compositions are prepared using already known techniques available to the person skilled in the art, who is capable of using known machinery and apparatus and the suitable production methods.

In addition to comprising a mixture of bacterial strains selected from among the 5-strain mixture or the 4-strain mixture or the 3-strain mixture (a) or (b), the compositions can contain elements or substances with antioxidant activity as mentioned above, in an amount by weight comprised from 0.0001% to 30% relative to the weight of the final composition, depending on the concentration of substances with antioxidant activity and/or of the recommended daily allowance (RDA), where defined.

Selenium can be present in the form of sodium selenate, L-selenomethionine, sodium selenite, sodium acid selenite and selenious acid, as well as in the form of selenium-enriched microorganisms, e.g. yeasts, in an amount by weight comprised from 0.0005% to 0.005% relative to the weight of the final composition, in any case sufficient to contribute a quantity of selenium preferably comprised from 10 µg to 150 µg.

Advantageously, the selenium is present in the form of selenium internalized in cells of probiotic bacteria.

In addition to comprising a mixture of bacterial strains selected from among the 5-strain mixture or the 4-strain mixture or the 3-strain mixture (a) or (b), the composition can moreover contain at least one bacterial strain deposited by the company BIOMAN S.r.l., Via Alfieri 18, 10100 Turin, Italy, namely:
- *Lactobacillus buchneri* LB26BM, deposited with the DSMZ on 05/04/2004 and having the deposit number DSM 16341, and/or
- *Lactobacillus ferintoshensis* LB6BM, deposited with the DSMZ on 17/01/2004 and having the deposit number DSM 16144, and/or
- *Lactobacillus reuteri* LB2BM, deposited with the DSMZ on 17/01/2004 and having the deposit number DSM 16143.

The compositions can further contain at least one other strain selected from among those which follow.

The following bacterial strains were deposited by the company Probiotical SpA Via Mattei, 3 -28100 Novara (NO) Italy with the DSMZ-Deutsche *Sammlung von Mikro-organismen und Zellkulturen* GmbH, Germany on 16.02.2012, in accordance with the Budapest Treaty:
i) *Lactobacillus johnsonii* DLLJO 01 = DSM 25680
ii) *Lactobacillus rhamnosus* DLLR 07 = DSM 25681
iii) *Lactobacillus rhamnosus* DLLR 08 = DSM 25682
iv) *Lactobacillus reuteri* DLLRE 07 = DSM 25683
v) *Lactobacillus reuteri* DLLRE 08 = DSM 25684
vi) *Lactobacillus reuteri* DLLRE 09 = DSM 25685
vii) *Bifidobacterium infantis* BI 03 = DSM 25709
viii) *Lactobacillus plantarum* LP 09 = DSM 25710

The present invention relates to a process for preparing cultures of bacteria belonging to the species *Bifidobacterium longum,* wherein said process comprises a step of synergistic co-culture of individual bacterial strains belonging to the species *B*. *longum* in which at least two bacterial strains said strains are selected from among those indicated as (1), (2) and (4) - are made to grow and replicate together in a same culture substrate.

The pharmaceutical or cosmetic composition or medical device or supplement product or food composition (in short, the compositions) comprises at least two bacterial strains. Said strains are obtained via a synergistic "co-culture" of bacteria of the same species *B*. *longum;* preferably, said strains are selected from among those indicated as (1) to (14). Said at least two bacterial strains are obtained through a process of fermentation that includes a step of bacterial co-culture in which said at least two bacterial strains are made to grow and replicate together in a same culture substrate. The compositions are for use in a treatment for helping to prolong life of a human being; or for use in a treatment for slowing down and/or combating and/or reducing the biological processes of physical and skin aging; or for use in a treatment for slowing down and/or combating and/or reducing the biological processes of aging which lead to loss of memory and/or the ability to concentrate.

The pharmaceutical or cosmetic composition or medical device or supplement product or food composition (in short, the compositions) comprises at least two bacterial strains belonging to the species *B*. *longum;* preferably, said strains are selected from among those indicated as (1) to (14), obtained via a "bacterial co-culture", for use in a treatment to inhibit the production of bacteroides by means of a non-specific mechanism of inhibition which involves the production of metabolites by said bacteria and/or by means of a specific mechanism which involves the production of bacteriocins by said bacteria; or for use in a treatment to stimulate the production of butyric *Clostridia* capable of producing butyrates to combat the onset of colitis, ulcerative colitis, inflammation of the intestine or of the gastrointestinal tract or Crohn's disease.

The pharmaceutical or cosmetic composition or medical device or supplement product or food composition (in short, the compositions) comprises at least two bacterial strains belonging to the species *B. longum*; preferably, said strains are selected from among those indicated as (1) to (14), obtained via "co-culture", for use in a treatment to inhibit and/or reduce Enterobacteria, belonging to the *Enterobacteriaceae* family and present in microbiota.

The pharmaceutical or cosmetic composition or medical device or supplement product or food composition (in short, the compositions) comprises at least two bacterial strains belonging to the species *B*. *longum;* preferably, said strains are selected from among those indicated as (1) to (14), obtained via "co-culture", for use in an antioxidant or immunomodulating treatment with the production of cytokines.

Said composition, preferably, can further comprise two or three or four or five or six bacterial strains in accordance with those indicated as (1) to (14) and obtained via "co-culture"; preferably in association with N-acetylcysteine or a substance based on N-acetylcysteine or a derivative thereof; preferably in association with selenium in the form of sodium selenate, L-selenomethionine, sodium selenite, sodium acid selenite, selenious acid or selenium internalized in cells of bacteria selected from the group comprising or, alternatively, consisting of:
- *Lactobacillus buchneri* LB26BM, deposited with the DSMZ on 05/04/2004 and having the deposit number DSM 16341, and/or
- *Lactobacillus ferintoshensis* LB6BM, deposited with the DSMZ on 17/01/2004 and having the deposit number DSM 16144, and/or
- *Lactobacillus reuteri* LB2BM, deposited with the DSMZ on 17/01/2004 and having the deposit number DSM 16143.

Said composition, preferably, can further comprise at least two bacterial strains from (1) to (14), in association with at least one other bacterial strain selected from the group comprising:
i) *Lactobacillus johnsonii* DLLJO 01 = DSM 25680
ii) *Lactobacillus rhamnosus* DLLR 07 = DSM 25681
iii) *Lactobacillus rhamnosus* DLLR 08 = DSM 25682
iv) *Lactobacillus reuteri* DLLRE 07 = DSM 25683
v) *Lactobacillus reuteri* DLLRE 08 = DSM 25684
vi) *Lactobacillus reuteri* DLLRE 09 = DSM 25685
vii) Bifidobacterium infantis BI 03 = DSM 25709
viii) Lactobacillus plantarum LP 09 = DSM 25710

The compositions can further contain at least one other strain selected from among those indicated in Tables A.

### Experimental part

The intestinal microbiota of 14 people having an age comprised from 100 to 104 years (group of centenarian subjects) was examined, along with the intestinal microbiota of 10 people having an age comprised from 27 to 54 years (group of adult subjects). Bacteria belonging to the following groups were analyzed: *Enterococcus*, *Staphylococcus*, *Lactobacillus*, *Bifidobacterium*, *Clostridia*, *Bacteroides*, *yeasts.*

Table 1 shows, in a statistically significant manner: total anaerobes, Enterobacteriaceae, Bifidobacteria, Bacteroides and Clostridia. One month before the start of the clinical study (collection of samples), none of the subjects took antibiotics, anti-constipation or anti-diarrhoea drugs, proton pump inhibitors or products containing probiotic bacteria. Subjects with chronic intestinal diseases or metabolic diseases (diabetes, obesity, diseases connected to malabsorption) were excluded from the clinical study. Fresh faecal samples were collected in sterile tubes and stored at -80°C for a maximum period of one month before undergoing analysis. Each subject provided two samples in two successive weeks. The main value of the two samples was evaluated. The samples were homogenized in a sterile peptone salt solution (casein digested with enzymes 1 g/l, sodium chloride 8.5 g/l). A serial dilution (10 times) was carried out and 100 µl of three appropriate dilutions were plated in the following media: Tryptic Soy Agar (TSA) + 5% blood for the total aerobes; Schaedler agar (SCH) + 5% blood for the total anaerobes; De Man Rogosa and Sharpe agar (MRS) for Lactobacilli; Bifidobacterium Selective Medium (BSM) for Bifidobacteria; MacConkey agar (MC) for Enterobacteriaaceae; Slanetz agar (SZ) for enterococci; Schaedler + Kanamicina/Vancomicina (KV) for Bacteroides: Clostrisel agar (CLOS) for Clostridia; and Sabouraud agar (SAB) + Cloramphenicol 500 mg/l for yeasts. The plates were incubated as follows: TSA at 37°C for 24 hours in 10% CO₂-enriched air; MC, SZ and SAB at 37°C under anaerobiosis for 24 hours, 24 and 48 hours, respectively; SCH, MRS, BSM, KV and CLOS at 37°C under anaerobiosis for 72 hours. After incubation the visible colonies were initially identified according to their morphology, staining for Gram-positive, staining for Gram-negative and catalase and oxidase testing. They were subsequently cultured under selected conditions. Then the colonies were counted and the number of microorganisms was expressed as colony forming units per gram of wet faeces (CFU/g). The detection level was 10² CFU/g. The Bifidobacteria and Lactobacilli, the colonies of which were previously counted and differentiated according to their morphology, were further identified on a species level by 16S rDNA sequencing using the method technically known as Pyrosequencing. The bacterial DNA was extracted from a pure culture. Suspensions of about 10¹⁰ CFU/ml were heated at 100°C for 10 minutes and centrifuged at 18000 rpm for 2 minutes. The supernatants were stored at -20°C pending analysis. The bacterial 16S rDNA was sequenced and compared with the online database described by Jonasson et al.; (Classification, idenfificafion and subtyping of bacteria based on pyrosequencing and signature matching of 16S rDNA fragments - APMIS2002; 110:263-272). The differences between the bacterial counts determined in the 14 centenary subjects and in the 10 younger adult subjects were statistically analyzed (Wilcoxon-Mann-Whitney Test). The results are shown in Table 2.

**Table 1: Main bacterial count (main value ± SD log10 per gram of wet faeces) for the 14 centenary subjects and for the 10 younger adult subjects.**

| | **Centenaries (Age 100-104 years)** | **Adults (Age 24-57 years)** | |
|---|---|---|---|
| **Total Aerobes** | 7.7 ± 0.8 | 8.2 ± 1.2 | n.s. |
| **Total Anaerobes** | 8.3 ± 0.6 | 9.1 ± 0.2 | p < 0.05 |
| **Enterobacteriaceae** | 4.2 ± 1.7 | 6.6 ± 1.4 | p < 0.05 |
| **Enterococci** | 6.3 ± 1.8 | 6.9 ± 1.4 | n.s. |
| **Staphylococci** | 3.6 ± 1.2 | 4.9 ± 1.2 | n.s. |
| **Lactobacilli** | 4.8 ± 2.3 | 5.4 ± 1.2 | n.s. |
| **Bifidobacteria** | 6.7 ± 1.4 | 8.3 ± 0.7 | p < 0.05 |
| **Bacteroides** | 5.9 ± 1.3 | 7.7 ± 0.8 | p < 0.05 |
| **Clostridia** | 4.1 ± 2.3 | 2.0 ± 0.0* | p < 0.05 |
| **Yeasts** | 2.5 ± 0.9 | 2.0 ± 0.0* | n.s. |

| | | | |
|---|---|---|---|
| n.s. = not statistically significant *the counts lower than the detection limit (10² CFU/g) were considered as 90 CFU/g. | | | |

**Table 2: Bacterial species belonging to the genera Lactobacillus and Bifidobacterium isolated from centenaries and younger adult subjects.**

| **Species** | **Number of subjects and count intervals** | |
|---|---|---|
| | **Centenaries (100-104 years)** | **Adults (24-57 years)** |
| *Lactobacillus reuteri* | 3 (10⁵ - 10⁷) | 0 |
| *Lactobacillus johnsonii* | 2 (10⁶ - 10⁷) | 0 |
| *Lactobacillus rhamnosus* | 2 (10³ - 10⁴) | 0 |
| *Lactobacillus sakei* | 2 (10⁵) | 2 (10⁵ - 10⁶) |
| *Lactobacillus casei* | 1 (10⁴) | 2 (10⁴ - 10⁵) |
| *Lactobacillus fermentum* | 0 | 1 (10³) |
| *Lactobacillus plantarum* | 0 | 2 (10⁴ - 10⁵) |
| *Lactobacillus paralimentarius* | 0 | 1 (10⁴) |
| *Lactobacillus gasseri* | 0 | 2 (10³ - 10⁴) |
| *Bifidobacterium longum* | 14 (10³ - 10⁸) | 3 (10⁸) |
| *Bifidobacterium adolescentis* | 2 (10⁴ - 10⁸) | 6 (10⁷ - 10⁸) |
| *Bifidobacterium bifidum* | 1 (105) | 4 (10⁷) |
| *Bifidobacterium pseudocatenulatum* | 0 | 4 (10⁷) |
| *Bifidobacterium catenulatum* | 0 | 2 (10⁷-10⁸) |

An evaluation was also made of a co-culture of several strains of the same species of *Bifidobacterium longum* isolated from centenary subjects, cultured individually (one at a time) and co-cultured in binary 14 association (two at a time) or ternary association (three at a time). The present study refers to the 5 different biotypes of *Bifidobacterium longum* shown in Table 3 below.

**Table 3**

| **Code** | **Strain collection ID** | **Deposit number** |
|---|---|---|
| DLBL 07 | 1820 | DSM 25669 |
| DLBL 08 | 1823 | DSM 25670 |
| DLBL 09 | 1821 | DSM 25671 |
| DLBL 10 | 1824 | DSM 25672 |
| DLBL 11 | 1825 | DSM 25673 |

The aforesaid strains were taken from a freezer at -80°C and thawed at a temperature of 37°C for 5 minutes. Thereafter they were transplanted - both individually at a percentage of 3% and in a mixture containing 0.6% of each strain (so as to obtain a total inoculum of 3%) - into the TPY culture broth for bifidobacteria (according to Scardovi), whose composition is described below:

| | |
|---|---|
| Trypticase peptone | 10 g |
| Soytone peptone | 5 g |
| Glucose | 10 g |
| Yeast extract | 5 g |
| Tween 80 | 1 ml |
| K₂HPO₄ | 2 g |
| MgCl₂ | 0.50 g |
| ZnSO₄ | 0.25 g |
| CaCl₂ | 0.15 g |
| Distilled water | 1000 ml |
| 5%* L(-)cysteine hydrochloride solution sterilized with a 0.20 µm filter | |
| (2.5g in 50 ml of water) | 10 ml |

The medium was previously reconstituted and sterilized at 121°C for 15 minutes. After sterilization the pH was equal to 6.60 (25°C). At the time of use, the 5% L(-)cysteine hydrochloride solution was added aseptically to the base medium.

The 5 strains and the mixture thereof was incubated at a temperature of 37°C under anaerobic conditions using Gas-pak jars containing AnaeroGen Oxoid (code AN0035A) anaerobiosis systems. After 5 hours of incubation the bifidobacteria cultures were cooled and subjected to testing for pH and optical density at a wavelength of 560 nm and a viable cell count according to the ISO 29981:2010 (E) IDF 220:2010(E) method with TOS-propionate agar medium + mupirocin as described below:

| | |
|---|---|
| Trypticase peptone | 10 g |
| Yeast extract | 1.0 g |
| K₂HPO₄ | 4.8 g |
| KH₂PO₄ | 3 g |
| (NH₄)₂SO₄ | 3 g |
| MgSO•7H₂O | 0.20 g |
| (R)-cysteine•HCl•H₂O | 0.5 g |
| Sodium propionate | 15.0 g |
| TOS* | 10.0 g |
| Distilled water | 965 ml |

| | |
|---|---|
| *TOS: mixture of galactose and glucose obtained by enzymatic hydrolysis of lactose using a β-galactosidase from *Aspergillus oryceae.* | |

The above-described broth was reconstituted and sterilized at 121°C for 15 minutes. Prior to use, a filter-sterilized mupirocin solution was added so as to obtain a final antibiotic concentration in the medium of 50 mg/litre.

### Results

The results obtained refer to the strains cultured individually and in binary and ternary mixtures (after 5 hours of incubation), see Table 4.

**Table 4**

| **Code** | **pH** | **OD** | **Viable cells** |
|---|---|---|---|
| DLBL 07 | 5.54 | 0.239 | 27 million/ml |
| DLBL 08 | 5.67 | 0.316 | 38 million/ml |
| DLBL 09 | 5.66 | 0.178 | 21 million/ml |
| DLBL 10 | 5.53 | 0.288 | 29 million/ml |
| DLBL 11 | 5.82 | 0.385 | 47 million/ml |
| Binary mixtures (DLBL 07, and/or DLBL 08, and/or DLBL 09, and/or DLBL 10, and/or DLBL 11) | 5.40 | 0.390 | From 40 to 45 million/ml |
| Binary mixtures of DLBL 07 and DLBL 08 | 5.50 | 0.392 | 41 million/ml |
| Binary mixtures of DLBL 08 DLBL 10 | 5.40 | 0.395 | 43 million/ml |
| Binary mixtures of DLBL 10 and DLBL 11 | 5.45 | 0.390 | 45 million/ml |
| Ternary mixture of DLBL 07 and DLBL 08 and DLBL 09 | 5.35 | 0.410 | 45 million/ml |
| Ternary mixture of DLBL 08 and DLBL 10 and DLBL 11 | 5.45 | 0.395 | 49 million/ml |
| Ternary mixture of DLBL 09 and DLBL 10 and DLBL 11 | 5.50 | 0.400 | 47 million/ml |
| Ternary mixture of DLBL 07 and DLBL 08 and DLBL 11 | 5.40 | 0.405 | 48 million/ml |
| Mixture of 4 strains (DLBL 07 and DLBL 08 and DLBL 09 and DLBL 11) | 5.39 | 0.395 | 49 million/ml |
| Mixture of 5 strains (DLBL 07 and DLBL 08 and DLBL 09 and DLBL 10 and DLBL 11) | 5.41 | 0.405 | 53 million/ml |

As can be noted, the viable cell counts of the bifidobacteria cultured individually are lower than those of the binary, ternary and quaternary mixtures thereof and that of the 5-strain mixture. The average of the values referring to individual strains is 32.4 million/ml, whereas in the binary mixture it is comprised from 40 to 45 million/ml; in the ternary and quaternary mixtures it is comprised from 45 to 49 million/ml. In the 5-strain mixture 53 million/ml were found, equal to a 63% increase. The better results obtained in terms of pH, optical density and viable cell count in the binary, ternary and 5-strains mixtures of co-cultured bifidobacteria are ascribable to a synergistic effect among the strains.

### Experimental part: immunomodulating properties.

An evaluation was made of the immunomodulating properties of the strains *Bifidocterium longum* DLBL 07 (DSM 25669), *Bifidocterium longum* DLBL 08 (DSM 25670), *Bifidocterium longum* DLBL 09 (DSM 25671), *Bifidocterium longum* DLBL 10 (DSM 25672) and *Bifidocterium longum* DLBL 11 (DSM 25673), all having a bacterial concentration of 1x10⁹ CFU/g. The study was carried out on the individual strains and on the 5-strain mixture (ratio by weight [1:1:1:1:1]), 4-strain mixture (ratio by weight [1:1:1:1]) and 3-strain mixtures (a) and (b) (ratio by weight [1:1:1]). More specifically, the analysis was conducted on culture supernatants after different stimulation times in order to analyze both the cytokines involved in the innate immunity and those responsible for the acquired immunity.

### i) Bacterial cultures and growth conditions

The strains were cultured in Man Rogosa Sharpe (MRS) medium in a temperature-controlled bath at 37°C. For the immunomodulation experiments, after about 16 hours of growth, the bacteria were subcultured for 6 hours under the above-mentioned conditions in order to reach the exponential growth stage.

They were then washed twice with sterile phosphate buffered saline (PBS, pH 7.2); the physiological state and number of cells was determined by flow cytometry using a commercial kit, "Cell Viability Kit with liquid beads", sold by the company Becton Dickinson, and following the instructions provided by the manufacturer. The cells were then brought to the optimal concentration established in preliminary experiments and used in subsequent tests.

### ii) Separation of peripheral blood mononuclear cells

Peripheral blood mononuclear cells (PBMCs) were separated by density gradient centrifugation. For this purpose, in each experiment use was made of 20 ml of buffy coats obtained from healthy donors at the Immuno-transfusion Department of the Borgomanero Hospital, with an average yield of 200 x 10⁶ PBMC/buffy. The amount of separated cells was determined in Bürker cell counting chambers using Turk's stain, which enables a distinction to be made between mononuclear cells and polymorphonuclear cells.

The cells were brought to a concentration of 2 x 10⁶ cells/ml in RPMI-1640 (Invitrogen) culture medium supplemented with 10% heat-inactivated foetal calf serum (FCS, Gibco), 1% glutamine and 25mM HEPES.

### iii) PBMC stimulation with bifidobacteria

After separation, the PBMCs were stimulated with the bacterial strains for 1 and 5 days. The internal controls of each individual experiment were represented by:
- Negative control:: PBMCs alone
- 1-day control:: PBMCs stimulated with 1 µg/ml Lipopolysaccharides (LPS; Escherichia coli, serotype 055:B5, Sigma Chemicals Co., St. Louis, MO).
- 5-day control:: PBMCs stimulated with 1 µg/ml Phytohaemagglutinin (PHA-P; Sigma Chemicals Co., St. Louis, MO).

At different times of analysis, the cultures were centrifuged at 1500 rpm for 10 minutes. The supernatants were removed and stored at -20°C until the time of analysis. The cells were used for subsequent tests.

### iv) Cytokine assay

The concentration of cytokines present in the culture supernatants was determined by E.L.I.S.A. **(Enzyme-Linked Immunoabsorbent Assay). More specifically, the "ELISA Module set" by the company Bender MedSystems was used to assay the cytokines IL-10 and IFN-gamma. Fig. 1 shows the mean cytokine secretion ±** S.E.M. of 4 independent trials. The statistical significance was calculated using a Student's *t*-test*.* Values of p < 0.05, calculated relative to the baseline value (unstimulated PBMCs), are to be considered statistically significant. The production of the cytokine IL-10 was evaluated in the culture supernatants after 1 day of stimulation. The production of IFN-gamma was evaluated in the culture supernatants after 5 days of stimulation.

### v) Statistical analysis

The statistical analysis was performed using a Student's *t*-test for paired data. A value of p < 0.05 was considered significant.

### vi) Results

### Cytokine secretion

The different spectrum of cytokines secreted by cellular subpopulations involved in the immune response plays an important role in the choice of the type of effector system which must be used in response to a particular antigen provocation. T-lymphocytes are the main effectors and regulators of cell-mediated immunity. In response to an antigen or a pathogenic agent, T-cells synthesize and secrete a variety of cytokines which are needed for the growth and differentiation of and as activation factors for other immunocompetent cells.

In order to investigate whether the bacterial strains studied induced a different secretion of cytokines by PBMCs, the cells were activated for 1 and 5 days. The amount of cytokines (IFN-gamma and IL-10) released in the culture supernatants was determined by E.L.I.S.A.

### Cytokines with a prevalent pro-inflammatory action

In the present study an evaluation was made of the induction of the cytokine IFN-gamma, as the most representative of the cytokines having a prevalently pro-inflammatory action. As can be seen in figure 1, all the bacterial strains studied induced an increase in the secretion of IFN-gamma, as compared to the baseline conditions. Statistical significance was obtained following stimulation with the strains DLBL 07, DLBL 08 and DLBL 09. Stimulation with the 5-strain mixture (MIX 5) did not bring about a further increase in IFN-gamma secretion compared to what was obtained with some individual strains (Figure 1).

### Cytokines with a prevalent immunoregulating action

In the present study an evaluation was made of the induction of the cytokine IL-10, as the most representative of the cytokines having a prevalently immunoregulating action. As can be seen in figure 1, all the bacterial strains studied induced an increase in the secretion of the cytokine analyzed, as compared to the baseline conditions. Statistical significance was not achieved only in the presence of the strain DLBL 10.

Stimulation with the 5-strain mixture (MIX 5) did not bring about a further increase in IL-10 secretion compared to what was obtained with some individual strains (Figure 1).

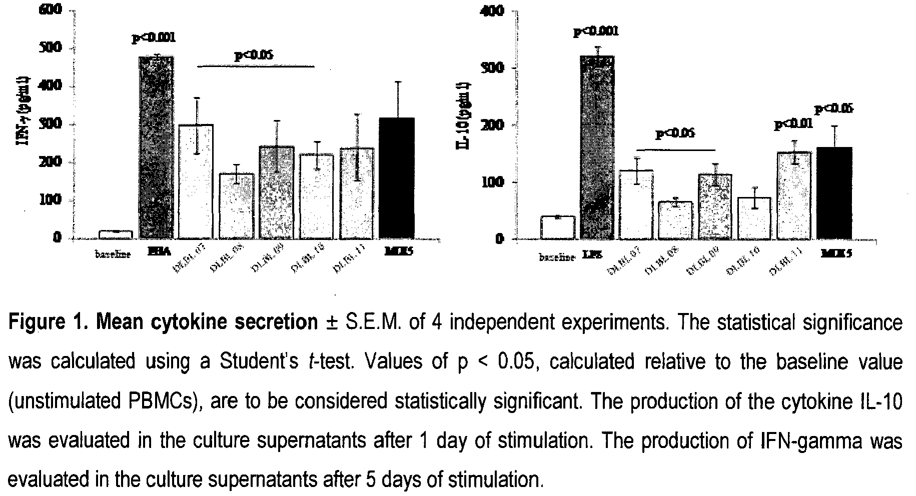

In the present study, the analysis of the individual cytokines secreted demonstrated the ability of the bacterial strains to induce an increase both in the pro-inflammatory cytokine IFN-gamma and in the anti-inflammatory/regulating cytokine IL-10.

In order to better understand the actual immunomodulating action of the bacterial strains, the ratio between the cytokine with a prevalent pro-inflammatory action and the one with a prevalent immunoregulating action was analyzed. Intestinal colonization by the bacteria with specific pro-Th1 properties, such as the strains studied on their own or in mixtures, could be an additional factor in all those pathologies which are characterized by a shift in the Th1/Th2 balance toward a pronounced Th2-type profile, such as allergic diseases (asthma, allergic rhinitis and atopic eczema) 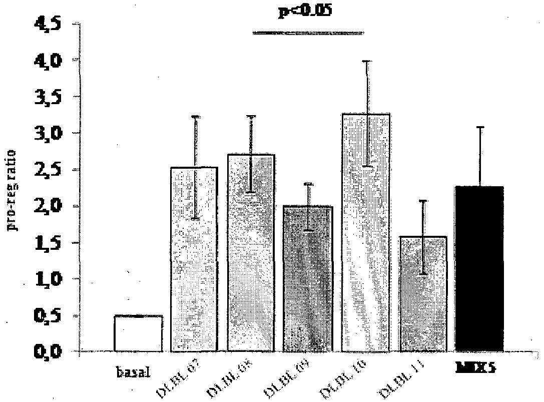 and autoimmune diseases caused by autoantibodies (systemic Lupus erythematosus and some forms of thyroiditis). The allergic reaction is in fact characterized by a shift in the Th1/Th2 balance toward a pronounced Th2-type profile, with production of the cytokines IL-4, IL-5, IL-9, and IL-13.

**Table A**

| **No.** | **Nome** | **Comm code** | **Deposit institution** | **Deposit number** | **Deposit date** | **Owner** |
|---|---|---|---|---|---|---|
| **1** | *Lactobacillus casei* | LF1i | CNCM I.P. | I-785 | 21.07.1988 | Anidral Srl |
| **2** | *Lactobacillus gasseri* | LF2i | CNCM I.P. | I-786 | 21.07.1988 | Anidral Srl |
| **3** | *Lactobacillus crispatus* | LF3i | CNCM I.P. | I-787 | 21.07.1988 | Anidral Srl |
| **4** | *Lactobacillus fermentum* | LF4i | CNCM I.P. | I-788 | 21.07.1988 | Anidral Srl |
| **5** | *Lactobacillus fermentum* | LF5 | CNCM I.P. | I-789 | 21.07.1988 | Anidral Sri |
| **6** | *Lactobacillus casei ssp. pseudoplantarum* | LFH i | CNCM I.P. | I-790 | 21.07.1988 | Anidral Srl |
| **7** | *Streptococcus thermophilus* B39 | | BCCM LMG | LMG P-18383 | 5.05.1998 | Anidral Srl |
| **8** | *Streptococcus thermophilus* T003 | | BCCM LMG | LMG P-18384 | 5.05.1998 | Anidral Srl |
| **9** | *Lactobacillus pentosus* 9/1 ei | | BCCM LMG | LMG P-21019 | 16.10.2001 | Mofin Srl |
| **10** | *Lactobacillus plantarum* 776/1 bi | LP 02 | BCCM LMG | LMG P-21020 | 16.10.2001 | Mofin Srl |
| **11** | *Lactobacillus plantarum* 476LL 20 bi | LP 01 | BCCM LMG | LMG P-21021 | 16.10.2001 | Mofin Srl |
| **12** | *Lactobacillus plantarum* PR ci | | BCCM LMG | LMG P-21022 | 16.10.2001 | Mofin Srl |
| **13** | *Lactobacillus plantarum* 776 /2 hi | | BCCM LMG | LMG P-21023 | 16.10.2001 | Mofin Srl |
| **14** | *Lactobacillus casei* ssp. *paracasei* 181A/3 aiai | LPC00 | BCCM LMG | LMG P-21380 | 31.01.2002 | Anidral Srl |
| **15** | *Lactobacillus* belonging to the *acidophilus* group 192A/1 aiai | LA 02 | BCCM LMG | LMG P-21381 | 31.01.2002 | Anidral Srl |
| **16** | *Bifidobacterium longum* 175A/1 aiai | | BCCM LMG | LMG P-21382 | 31.01.2002 | Anidral Srl |
| **17** | *Bifidobacterium breve* 195A/1 aici | | BCCM LMG | LMG P-21383 | 31.01.2002 | Anidral Sri |
| **18** | *Bifidobacterium lactis* 32A/3 aiai | BS 01 | BCCM LMG | LMG P-21384 | 31.01.2002 | Anidral Srl |
| **19** | *Lactobacillus plantarum* 501/2 gi | COAKTIV | BCCM LMG | LMG P-21385 | 31.01.2002 | Mofin Srl |
| **20** | *Lactococcus lactis* ssp. *lactis* 501/4 ci | | BCCM LMG | LMG P-21388 | 31.01.2002 | Mofin Srl |
| **21** | *Lactococcus lactis* ssp. *lactis* 501/4 hi | | BCCM LMG | LMG P-21387 | 15.03.2002 | Mofin Srl |
| **22** | *Lactococcus lactis* ssp. *lactis* 501/4 ci | | BCCM LMG | LMG P-21388 | 31.01.2002 | Mofin Srl |
| **23** | *Lactobacillus plantarum* 501/4 li | | BCCM LMG | LMG P-21389 | 15.03.2002 | Mofin Srl |
| **24** | *Lactobacillus acidophilus* | LA08 | BCCM LMG | LMG P-26144 | 03.11.2010 | Probiotical SpA |
| **25** | *Lactobacillus paracasei ssp. Paracasei* | LPC10 | BCCM LMG | LMG P-26143 | 03.11.2010 | Probiotical SpA |
| **26** | *Streptococcus thermophilus* | GB1 | DSMZ | DSM 16506 | 18.06.2004 | Anidral Srl |
| **27** | *Streptococcus thermophilus* | GB5 | DSMZ | DSM 16507 | 18.06.2004 | Anidral Srl |
| **28** | *Streptococcus thermophilus* | Y02 | DSMZ | DSM 16590 | 20.07.2004 | Anidral Sri |
| **29** | *Streptococcus thermophilus* | Y03 | DSMZ | DSM 16591 | 20.07.2004 | Anidral Srl |
| **30** | *Streptococcus thermophilus* | Y04 | DSMZ | DSM 16592 | 20.07.2004 | Anidral Srl |
| **31** | *Streptococcus thermophilus* | YO5 | DSMZ | DSM 16593 | 20.07.2004 | Anidral Srl |
| **32 =56** | *Bifidobacterium adolescentis* | BA 03 | DSMZ | DSM 16594 | 21.07.2004 | Anidral Srl |
| **33** | *Bifidobacterium adolescentis* | BA 04 | DSMZ | DSM 16595 | 21.07.2004 | Anidral Srl |
| **34** | *Bifidobacterium breve* | BR 04 | DSMZ | DSM 16596 | 21.07.2004 | Anidral Srl |
| **35** | *Bifidobacterium pseudocatenulatum* | BP 01 | DSMZ | DSM 16597 | 21.07.2004 | Anidral Srl |
| **36** | *Bifidobacterium pseudocatenulatum* | BP 02 | DSMZ | DSM 16598 | 21.07.2004 | Anidral Srl |
| **37** | *Bifidobacterium longum* | BL 03 | DSMZ | DSM 16603 | 20.07.2004 | Anidral Srl |
| **38** | *Bifidobacterium breve* | BR 03 | DSMZ | DSM 16604 | 20.07.2004 | Anidral Srl |
| **39** | *Lactobacillus casei* ssp. *rhamnosus* | LR 04 | DSMZ | DSM 16605 | 20.07.2004 | Anidral Srl |
| **40** | *Lactobacillus delbrueckii* ssp. *bulgaricus* | LDB 01 | DSMZ | DSM 16606 | 20.07.2004 | Anidral Srl |
| **41** | *Lactobacillus delbrueckii* ssp. *bulgaricus* | LDB 02 | DSMZ | DSM 16607 | 20.07.2004 | Anidral Srl |
| **42** | *Staphylococcus xylosus* | SX01 | DSMZ | DSM 17102 | 01.02.2005 | Anidral Srl |
| **43 = 57** | *Bifidobacterium adolescentis* | BA 02 | DSMZ | DSM 17103 | 01.02.2005 | Anidral Srl |
| **44** | *Lactobacillus plantarum* | LP 07 | DSMZ | DSM 17104 | 01.02.2005 | Anidral Srl |
| **45** | *Streptococcus thermophilus* | YO8 | DSMZ | DSM 17843 | 21.12.2005 | Anidral Srl |
| **46** | *Streptococcus thermophilus* | YO9 | DSMZ | DSM 17844 | 21.12.2005 | Anidral Srl |
| **47** | *Streptococcus thermophilus* | YO100 | DSMZ | DSM 17845 | 21.12.2005 | Anidral Srl |
| **48** | *Lactobacillus fermentum* | LF06 | DSMZ | DSM 18295 | 24.05.2006 | Anidral Srl |
| **49** | *Lactobacillus fermentum* | LF07 | DSMZ | DSM 18296 | 24.05.2006 | Anidral Srl |
| **50** | *Lactobacillus fermentum* | LF08 | DSMZ | DSM 18297 | 24.05.2006 | Anidral Srl |
| **51** | *Lactobacillus fermentum* | LF09 | DSMZ | DSM 18298 | 24.05.2006 | Anidral Srl |
| **52** | *Lactobacillus gasseri* | LGS01 | DSMZ | DSM 18299 | 24.05.2006 | Anidral Srl |
| **53** | *Lactobacillus gasseri* | LGS02 | DSMZ | DSM 18300 | 24.05.2006 | Anidral Srl |
| **54** | *Lactobacillus gasseri* | LGS03 | DSMZ | DSM 18301 | 24.05.2006 | Anidral Srl |
| **55** | *Lactobacillus gasseri* | LGS04 | DSMZ | DSM 18302 | 24.05.2006 | Anidral Srl |
| **56 32** | *Bifidobacterium adolescentis* EI-3 ***Bifidobacterium catenulatum sp.*/*pseudocatenulatum* EI-3I, ID** 09-255 | BA 03 | DSMZ | DSM 18350 | 15.06.2006 | Anidral Srl |
| **57 = 43** | *Bifidobacterium adolescentis* EI-15 | BA 02 | DSMZ | DSM 18351 | 15.06.2006 | Anidral Srl |
| **58** | *Bifidobacterium adolescentis* EI-18 ***Bifidobacterium animalis subsp.** lactis* EI-18, ID 09-256 | BA 05 | DSMZ | DSM 18352 | 15.06.2006 | Anidral Srl |
| **59** | *Bifidobacterium catenulatum* EI-20 | BC01 | DSMZ | DSM 18353 | 15.06.2006 | Anidral Sri |
| **60** | *Streptococcus thermophilus* FRai | MO1 | DSMZ | DSM 18613 | 13.09.2006 | Mofin Srl |
| **61** | *Streptococcus thermophilus* LB2bi | MO2 | DSMZ | DSM 18614 | 13.09.2006 | Mofin Srl |
| **62** | *Streptococcus thermophilus* LRci | MO3 | DSMZ | DSM 18615 | 13.09.2006 | Mofin Srl |
| **63** | *Streptococcus thermophilus* FP4 | MO4 | DSMZ | DSM 18616 | 13.09.2006 | Mofin Srl |
| **64** | *Streptococcus thermophilus* ZZ5F8 | MO5 | DSMZ | DSM 18617 | 13.09.2006 | Mofin Srl |
| **65** | *Streptococcus thermophilus* TEO4 | MO6 | DSMZ | DSM 18618 | 13.09.2006 | Mofin Srl |
| **66** | *Streptococcus thermophilus* S1ci | MO7 | DSMZ | DSM 18619 | 13.09.2006 | Mofin Srl |
| **67** | *Streptococcus thermophilus* 641 bi | MO8 | DSMZ | DSM 18620 | 13.09.2006 | Mofin Srl |
| **68** | *Streptococcus thermophilus* 277A/1ai | MO9 | DSMZ | DSM 18621 | 13.09.2006 | Mofin Srl |
| **69** | *Streptococcus thermophilus* 277A/2ai | MO10 | DSMZ | DSM 18622 | 13.09.2006 | Mofin Srl |
| **70** | *Streptococcus thermophilus* IDC11 | MO11 | DSMZ | DSM 18623 | 13.09.2006 | Mofin Sri |
| **71** | *Streptococcus thermophilus* ML3di | MO14 | DSMZ | DSM 18624 | 13.09.2006 | Mofin Srl |
| **72** | *Streptococcus thermophilus* TEO3 | MO15 | DSMZ | DSM 18625 | 13.09.2006 | Mofin Srl |
| **73** | *Streptococcus thermophilus* G62 | GG1 | DSMZ | DSM 19057 | 21.02.2007 | Mofin Srl |
| **74** | *Streptococcus thermophilus* G1192 | GG2 | DSMZ | DSM 19058 | 21.02.2007 | Mofin Srl |
| **75** | *Streptococcus thermophilus* GB18 | GG3 MO2 | DSMZ | DSM 19059 | 21.02.2007 | Mofin Srl |
| **76** | *Streptococcus thermophilus* CCR21 | GG4 | DSMZ | DSM 19060 | 21.02.2007 | Mofin Srl |
| **77** | *Streptococcus thermophilus* G92 | GG5 | DSMZ | DSM 19061 | 21.02.2007 | Mofin Srl |
| **78** | *Streptococcus thermophilus* G69 | GG6 | DSMZ | DSM 19062 | 21.02.2007 | Mofin Srl |
| **79** | *Streptococcus thermophilus* | YO 10 | DSMZ | DSM 19063 | 21.02.2007 | Anidral Srl |
| **80** | *Streptococcus thermophilus* | YO 11 | DSMZ | DSM 19064 | 21.02.2007 | Anidral Srl |
| **81** | *Streptococcus thermophilus* | YO 12 | DSMZ | DSM 19065 | 21.02.2007 | Anidral Srl |
| **82** | *Streptococcus thermophilus* | YO 13 | DSMZ | DSM 19066 | 21.02.2007 | Anidral Srl |
| **83** | *Weissella* ssp. WSP 01 | EX | DSMZ | DSM 19067 | 21.02.2007 | Anidral Srl |
| **84** | *Weissella* ssp. WSP 02 | EX | DSMZ | DSM 19068 | 21.02.2007 | Anidral Srl |
| **85** | *Lactobacillus* ssp. WSP 03 | EX | DSMZ | DSM 19069 | 21.02.2007 | Anidral Srl |
| **86** | *Lactobacillus plantarum* LP 09 | OY | DSMZ | DSM 19070 | 21.02.2007 | Anidral Srl |
| **87** | *Lactobacillus plantarum* LP 10 | OY | DSMZ | DSM 19071 | 21.02.2007 | Anidral Srl |
| **88** | *Lactococcus lactis* | NS 01 | DSMZ | DSM 19072 | 21.02.2007 | Anidral Srl |
| **89** | *Lactobacillus fermentum* | LF 10 | DSMZ | DSM 19187 | 20.03.2007 | Anidral Srl |
| **90** | *Lactobacillus fermentum* | LF 11 | DSMZ | DSM 19188 | 20.03.2007 | Anidral Srl |
| **91** | *Lactobacillus casei* ssp. *rhamnosus* | LR05 | DSMZ | DSM 19739 | 27.09.2007 | Anidral Sri |
| **92** | *Bifidobacterium bifidum* | BB01 | DSMZ | DSM 19818 | 30.10.2007 | Anidral Srl |
| **93** | *Lactobacillus delbrueckii* LD 01 | Lb | DSMZ | DSM 19948 | 28.11.2007 | Anidral Sri |
| **94** | *Lactobacillus delbrueckii* LD 02 | Lb | DSMZ | DSM 19949 | 28.11.2007 | Anidral Srl |
| **95** | *Lactobacillus delbrueckii* LD 03 | Lb | DSMZ | DSM 19950 | 28.11.2007 | Anidral Sri |
| **96** | *Lactobacillus delbrueckii* LD 04 | Lb | DSMZ | DSM 19951 | 28.11.2007 | Anidral Srl |
| **97** | *Lactobacillus delbrueckii* LD 05 | Lb | DSMZ | DSM 19952 | 28.11.2007 | Anidral Srl |
| **98** | *Bifidobacterium pseudocatenulatum* | B660 | DSMZ | DSM 21444 | 13.05.2008 | Probiotical SpA |
| **99** | *Lactobacillus acidophilus* | LA02 | DSMZ | DSM 21717 | 06.08.2008 | Probiotical SpA |
| **100** | *Lactobacillus paracasei* | LPC 08 | DSMZ | DSM 21718 | 06.08.2008 | Probiotical SpA |
| **101** | *Lactobacillus pentosus* | LPS 01 | DSMZ | DSM 21980 | 14.11.2008 | Probiotical SpA |
| **102** | *Lactobacillus rahmnosus* | LR 06 | DSMZ | DSM 21981 | 14.11.2008 | Probiotical SpA |
| **103** | *Lactobacillus delbrueckii* ssp. *Delbrueckii* | DSMZ 20074 | DSMZ | DSM 22106 | 10.12.2008 | Probiotical SpA |
| **104** | *Lactobacillus plantarum* | LP1 | DSMZ | DSM 22107 | 10.12.2008 | Probiotical SpA |
| **105** | *Lactobacillussalivarius* | LS01 | DSMZ | DSM 22775 | 23.07.2009 | Probiotical SpA |
| **106** | *Lactobacillus salivarius* | LS03 | DSMZ | DSM 22776 | 23.07.2009 | Probiotical SpA |
| **107** | *Bifidobacterium bifidum* | BB01 | DSMZ | DSM 22892 | 28.08.2009 | Probiotical SpA |
| **108** | *Bifidobacterium bifidum* | | DSMZ | DSM 22893 | 28.08.2009 | Probiotical SpA |
| **109** | *Bifidobacterium bifidum* | BB03 | DSMZ | DSM 22894 | 28.08.2009 | Probiotical SpA |
| **110** | *Bifidobacterium lactis* | BS05 | DSMZ | DSM 23032 | 13.10.2009 | Probiotical SpA |
| **111** | *Lactobacillus acidophilus* | LA 06 | DSMZ | DSM 23033 | 13.10.2009 | Probiotical SpA |
| **112** | *Lactobacillus brevis* | LBR01 | DSMZ | DSM 23034 | 13.10.2009 | Probiotical SpA |
| **113** | *Bifidobacterium animalis* ssp. *lactis* | BS06 | DSMZ | DSM 23224 | 12.01.2010 | Probiotical SpA |
| **114** | *Bifidobacterium longum* | BL04 | DSMZ | DSM 23233 | 12.01.2010 | Probiotical SpA |
| **115** | *Bifidobacferium longum* | BL05 | DSMZ | DSM 23234 | 12.01.2010 | Probiotical SpA |
| **116** | *Bifidobacterium bifidum* | MB 109 | DSMZ | DSM 23731 | 29.06.2010 | Probiotical SpA |
| **117** | *Bifidobacterium breve* | MB 113 | DSMZ | DSM 23732 | 29.06.2010 | Probiotical SpA |
| **118** | *Bifidobacterium lactis* | MB 2409 | DSMZ | DSM 23733 | 29.06.2010 | Probiotical SpA |
| **119** | *Lactobacillus reuteri* | LRE01 | DSMZ | DSM 23877 | 05.08.2010 | Probiotical SpA |
| **120** | *Lactobacillus reuteri* | LRE02 | DSMZ | DSM 23878 | 05.08.2010 | Probiotical SpA |
| **121** | *Lactobacillus reuteri* | LRE03 | DSMZ | DSM 23879 | 05.08.2010 | Probiotical SpA |
| **122** | *Lactobacillus reuteri* | LRE04 | DSMZ | DSM 23880 | 05.08.2010 | Probiotical SpA |
| **123** | *Lactobacillus paracasei* ssp. *paracasei* | LPC09 | DSMZ | DSM 24243 | 23.11.2010 | Probiotical SpA |
| **124** | *Lactobacillus acidophilus* | LA 07 | DSMZ | DSM 24303 | 23.11.2010 | Probiotical SpA |
| **125** | *Bifidobacterium bifidum* | BB04 | DSMZ | DSM 24437 | 04.01.2011 | Probiotical SpA |
| **126** | *Lactobacillus crispatus* | CRL 1251 | DSMZ | DSM 24438 | 04.01.2011 | Probiotical SpA |
| **127** | *Lactobacillus crispatus* | CRL 1266 | DSMZ | DSM 24439 | 04.01.2011 | Probiotical SpA |
| **128** | *Lactobacillus paracasei* | CRL 1289 | DSMZ | DSM 24440 | 04.01.2011 | Probiotical SpA |
| **129** | *Lactobacillus salivarius* | CRL 1328 | DSMZ | DSM 24441 | 04.01.2011 | Probiotical SpA |
| **130** | *Lactobacillus gasseri* | CRL 1259 | DSMZ | DSM 24512 | 25.01.2011 | Probiotical SpA |
| **131** | *Lactobacillus acidophilus* | CRL 1294 | DSMZ | DSM 24513 | 25.01.2011 | Probiotical SpA |
| **132** | *Lactobacillus salivarius* | LS04 | DSMZ | DSM 24618 | 02.03.2011 | Probiotical SpA |
| **133** | *Lactobacillus crispatus* | LCR01 | DSMZ | DSM 24619 | 02.03.2011 | Probiotical SpA |
| **134** | *Lactobacillus crispatus* | LCR02 | DSMZ | DSM 24620 | 02.03.2011 | Probiotical SpA |
| **135** | *Lacotbacillus acidophilus* | LA09 | DSMZ | DSM 24621 | 02.03.2011 | Probiotical SpA |
| **136** | *Lactobacillus gasseri* | LGS05 | DSMZ | DSM 24622 | 02.03.2011 | Probiotical SpA |
| **137** | *Lactobacillus paracasei* | LPC11 | DSMZ | DSM 24623 | 02.03.2011 | Probiotical SpA |
| **138** | *Bifidobacterium infantis* | BI 02 | DSMZ | DSM 24687 | 29.03.2011 | Probiotical SpA |
| **139** | *Bifidobacterium bifidum* | BB 06 | DSMZ | DSM 24688 | 29.03.2011 | Probiotical SpA |
| **140** | *Bifidobacterium longum* | BL 06 | DSMZ | DSM 24689 | 29.03.2011 | Probiotical SpA |
| **141** | *Bifidobacterium lactis* | BS 07 | DSMZ | DSM 24690 | 29.03.2011 | Probiotical SpA |
| **142** | *Bifidobacterium longum* | PCB133 | DSMZ | DSM 24691 | 29.03.2011 | Probiotical SpA |
| **143** | *Bifidobacterium breve* | B632 | DSMZ | DSM 24706 | 07.04.2011 | Probiotical SpA |
| **144** | *Bifidobacterium breve* | B2274 | DSMZ | DSM 24707 | 07.04.2011 | Probiotical SpA |
| **145** | *Bifidobacterium breve* | B7840 | DSMZ | DSM 24708 | 07.04.2011 | Probiotical SpA |
| **146** | *Bifidobacterium longum* | B1975 | DSMZ | DSM 24709 | 07.04.2011 | Probiotical SpA |
| **147** | *Lactobacillus salivarius* | DLV1 | DSMZ | DSM 25138 | 02.09.2011 | Probiotical SpA |
| **148** | *Lactobacillus Reuteri* | LRE05 | DSMZ | DSM 25139 | 02.09.2011 | Probiotical SpA |
| **149** | *Lactobacillus Reuteri* | LRE06 | DSMZ | DSM 25140 | 02.09.2011 | Probiotical SpA |
| **150** | *Lactobacillus Reuteri* | RC 14 | DSMZ | DSM 25141 | 02.09.2011 | Probiotical SpA |
| **151** | *Streptococcus thermophilus* | ST 10 | DSMZ | DSM 25246 | 19.09.2011 | Probiotical SpA |
| **152** | *Streptococcus thermophilus* | ST 11 | DSMZ | DSM 25247 | 19.09.2011 | Probiotical SpA |
| **153** | *Streptococcus thermophilus* | ST 12 | DSMZ | DSM 25282 | 20.10.2011 | Probiotical SpA |
| **154** | *Lactobacillus salivarius* | DLV8 | DSMZ | DSM 25545 | 12.01.2012 | Probiotical SpA |

## Claims

1. A process for preparing a mixture of cultures of bacteria belonging to the species *Bifidobacterium longum,* wherein said process comprises a step in which at least two bacterial strains belonging to the species *Bifidobacterium longum* selected from the group consisting of:
1) *Bifidobacterium longum* DLBL 07 = DSM 25669
2) *Bifidobacterium longum* DLBL 08 = DSM 25670
3) *Bifidobacterium longum* DLBL 10 = DSM 25672
are made to grow together simultaneously in a same culture substrate.

2. The process according to claim 1, wherein said bacterial mixture comprises three bacterial strains; preferably said three bacterial strains are:
- *Bifidobacterium longum* DLBL 07 = DSM 25669
- *Bifidobacterium longum* DLBL 08 = DSM 25670
- *Bifidobacterium longum* DLBL 11 = DSM 25673.

3. The process according to claim 1 or 2, wherein said bacterial mixture comprises four bacterial strains; preferably said four bacterial strains are:
- *Bifidobacterium longum* DLBL 07 = DSM 25669
- *Bifidobacterium longum* DLBL 08 = DSM 25670
- *Bifidobacterium longum* DLBL 09 = DSM 25671
- *Bifidobacterium longum* DLBL 11 = DSM 25673.

4. The process according to claim 1 or 2, wherein said bacterial mixture comprises five bacterial strains; preferably said five bacterial strains are:
- *Bifidobacterium longum* DLBL 07 = DSM 25669
- *Bifidobacterium longum* DLBL 08 = DSM 25670
- *Bifidobacterium longum* DLBL 09 = DSM 25671
- *Bifidobacterium longum* DLBL 10 = DSM 25672
- *Bifidobacterium longum* DLBL 11 = DSM 25673.

## Patentansprüche

1. Verfahren zur Herstellung einer Mischung von Bakterienkulturen der species *Bifidobacterium longum,* umfassend einen Schritt, in dem wenigstens zwei Bakterienstämme der species *Bifidobacterium longum* ausgewählt aus der Gruppe bestehend aus:
1) *Bifidobacterium longum* DLBL 07 = DSM 25669
2) *Bifidobacterium longum* DLBL 08 = DSM 25670
3) *Bifidobacterium longum* DLBL 10 = DSM 25672
miteinander gleichzeitig im selben Kultursubstrat zum Wachsen gebracht werden.

2. Verfahren gemäß Anspruch 1, wobei diese Bakterienmischung drei Bakterienstämme umfasst; und wobei diese drei Bakterienstämme vorzugsweise sind:
- *Bifidobacterium longum* DLBL 07 = DSM 25669
- *Bifidobacterium longum* DLBL 08 = DSM 25670
- *Bifidobacterium longum* DLBL 11 = DSM 25673.

3. Verfahren gemäß Anspruch 1 oder 2, wobei diese Bakterienmischung vier Bakterienstämme umfasst; und wobei diese vier Bakterienstämme vorzugsweise sind:
- *Bifidobacterium longum* DLBL 07 = DSM 25669
- *Bifidobacterium longum* DLBL 08 = DSM 25670
- *Bifidobacterium longum* DLBL 09 = DSM 25671
- *Bifidobacterium longum* DLBL 11 = DSM 25673.

4. Verfahren gemäß Anspruch 1 oder 2, wobei diese Bakterienmischung fünf Bakterienstämme umfasst; und wobei diese fünf Bakterienstämme vorzugsweise sind:
- *Bifidobacterium longum* DLBL 07 = DSM 25669
- *Bifidobacterium longum* DLBL 08 = DSM 25670
- *Bifidobacterium longum* DLBL 09 = DSM 25671
- *Bifidobacterium longum* DLBL 10 = DSM 25672
- *Bifidobacterium longum* DLBL 11 = DSM 25673.

## Revendications

1. Procédé de préparation d'un mélange de cultures de bactéries appartenant à l'espèce *Bifidobacterium longum,* dans lequel ledit procédé comprend une étape dans laquelle au moins deux souches bactériennes appartenant à l'espèce *Bifidobacterium longum* choisies dans le groupe constitué par :
1) *Bifidobacterium longum* DLBL 07 = DSM 25669
*2) Bifidobacterium longum* DLBL 08 = DSM 25670
*3) Bifidobacterium longum* DLBL 10 = DSM 25672
sont mises en croissance ensemble simultanément dans un même substrat de culture.

2. Procédé selon la revendication 1, dans lequel ledit mélange bactérien comprend trois souches bactériennes ; de préférence lesdites trois souches bactériennes sont :
- *Bifidobacterium longum* DLBL 07 = DSM 25669
- *Bifidobacterium longum* DLBL 08 = DSM 25670
- *Bifidobacterium longum* DLBL 11 = DSM 25673.

3. Procédé selon la revendication 1 ou 2, dans lequel ledit mélange bactérien comprend quatre souches bactériennes ; de préférence lesdites quatre souches bactériennes sont :
- *Bifidobacterium longum* DLBL 07 = DSM 25669
- *Bifidobacterium longum* DLBL 08 = DSM 25670
- *Bifidobacterium longum* DLBL 09 = DSM 25671
- *Bifidobacterium longum* DLBL 11 = DSM 25673.

4. Procédé selon la revendication 1 ou 2, dans lequel ledit mélange bactérien comprend cinq souches bactériennes ; de préférence lesdites cinq souches bactériennes sont :
- *Bifidobacterium longum* DLBL 07 = DSM 25669
- *Bifidobacterium longum* DLBL 08 = DSM 25670
- *Bifidobacterium longum* DLBL 09 = DSM 25671
- *Bifidobacterium longum* DLBL 10 = DSM 25672
- *Bifidobacterium longum* DLBL 11 = DSM 25673.
